# EUROPEAN PATENT SPECIFICATION

(11) **EP 2 030 963 B1**
(45) Date of publication and mention of the grant of the patent: **08.06.2011**
(21) Application number: 08168030.8
(22) Date of filing: 14.07.1998
(51) Int. Cl.: C07C 51/50, C07C 57/04, C07C 69/54, C07C 67/62, C07C 69/15, C07C 231/22, C07C 233/09, C07C 253/32, C07C 255/08, C07B 63/04, C07D 211/94

(54) **Derivatives of 1-oxyl-4-hydroxy-2,2,6,6-tetramethylpiperidine as polymerization inhibitors for (meth) acrylate monomers**
Derivate von 1-oxyl-4-hydroxy-2,2,6,6-Tetramethylpiperidin als Polymerisationsinhibitoren für (Meth)acrylatmonomere
Dérivés de 1-oxyl-4-hydroxy-2,2,6,6-tétraméthylpiperidine, utilisés comme inhibiteurs de polymérisation pour des monomères (meth)acrylates

(43) Date of publication of application: 04.03.2009
(62) Divisional of application: 98936423.7
(73) Proprietor: BASF SE, 67056 Ludwigshafen (DE)
(72) Inventor: Cunkle, Glen, Thomas, Stamford, CT 06903 (US); Gande, Matthew, Edward, Norwalk, CT 06851 (US); Seltzer, Raymond, New City, NY 10956 (US); Thompson, Thomas, Friend, Highland Mills, NY 10930 (US)

(56) References cited:
- EP-A- 0 697 386
- WO-A-98/02400
- WO-A-98/56746
- WO-A-99/05108
- DE-A- 19 510 184
- DE-A- 19 609 312
- US-A- 5 322 960
- CHEMICAL ABSTRACTS, vol. 104, no. 17, 28 April 1986 (1986-04-28), Columbus, Ohio, US; abstract no.: 148699w, BARACU I ET AL: "Potential anticancer agents. XXVI. Spin labeled nitrosoureas" page 677 XP002097007 & J. PRAKT. CHEM., vol. 327, no. 4, 1985, pages 667-74,

## Description

The instant invention pertains to selected derivatives of 1-oxyl-2,2,6,6-tetramethyl-4-hydroxypiperidine as inhibitors for preventing the premature polymerization of acrylic and methacrylic acids, their esters and amides, of vinyl acetate and of acrylonitrile in the presence of water.

Many of the industrially important ethylenically unsaturated monomers are highly susceptible to unwanted radical polymerization initiated either thermally or by adventitious impurities. Some examples of these monomers are acrylic and methacrylic acid, acrylate and methacrylate esters, acrylamide and methacrylamide, vinyl acetate and acrylonitrile. Premature polymerization may occur during manufacture, purification or storage of the monomer. Many of these monomers are purified by distillation. It is in this operation where premature polymerization is most likely to occur and to be the most troublesome. Methods to prevent or reduce the amount of such polymerization are thus highly desirable since the prevention or mitigation of such premature polymerization increases the yield of purified monomer and also insures against costly and potentially dangerous runaway polymerization in the plant.

Stable nitroxides are known in the art to be effective in preventing the premature radical polymerization of ethylenically unsaturated monomers. Some examples are seen in Japanese Hei 9-268138 which discloses the stabilization of styrene by 1-oxyl-2,2,6,6-tetramethyl-4-hydroxypiperidine and its lower alkyl ethers in the presence of nitrophenols. United States Patent Nos. 3,747,988 describes the use of 1-oxyl-2,2,6,6-tetramethyl-4-hydroxy-piperidine as a polymerization inhibitor for acrylonitrile in the presence of water and oxygen. United States Patent No. 3,488,338 discloses that 1-oxyl-2,2,6,6-tetramethyl-4-hydroxy-piperidine is an effective chain stopper in the aqueous polymerization of chloroprene. British Patent No. 1,127,127 describes the stabilization of neat acrylic acid by 1-oxyl-2,2,6,6-tetramethyl-4-hydroxypiperidine. Japanese Sho 60-36501 describes the stabilization of acrylate and methacrylate esters.

United States Patent Nos. 5,322,960 and 5,504,243 disclose the use of 1-oxyl-2,2,6,6-tetramethyl-4-hydroxypiperidine in preventing the polymerization of acrylic and methacrylic acids and their esters in the presence of water, but tout the great advantages of using said oxyl compound in combination with manganese acetate, or with hydroquinone and phenothiazine.

EP 178,168 teaches the use of 1-oxyl-2,2,6,6-tetramethyl-4-hydroxypiperidine or its reaction product with hexamethylene diisocyanate in stabilizing acrylic acid or methacrylic acid in the presence of water.

EP 791,573 discloses that the lower alkyl or aryl esters of 1-oxyl-2,2,6,6-tetramethyl-4-hydroxypiperidine are effective polymerization inhibitors alone or in combination with various coadditives for vinyl acetate in the presence of water.

Japanese Hei 5-320205 generically describes the use of 1-oxyl-2,2,6,6-tetramethyl-4-hydroxypiperidine, its lower alkyl ethers and lower alkanoic esters in preventing the polymerization of acrylic and methacrylic acids alone, but preferably in the presence of chelating agents for ferric salts, such as ethylenediaminetetraacetic acid. The 4-hydroxy, 4-methoxy and 4-acetoxy derivatives are specifically disclosed.

Japanese Hei 5-320217 teaches the stabilization of acrylic and methacrylic acids with 1-oxyl-2,2,6,6-tetramethyl-4-hydroxypiperidine, its lower alkyl ethers and lower alkanoic esters alone, but preferably in the presence of phenothiazine, an aromatic amine or phenol. The 4-hydroxy, 4-methoxy and 4-acetoxy derivatives are specifically disclosed.

German Application DE 195 10 184 A1 describes amide and formamide derivatives of 1-oxyl-2,2,6,6-tetramethyl-4-aminopiperidine as stabilizers for radically polymerizable monomers, but does not differentiate between aqueous and non-aqueous systems.

German Patent DE 196 09 312 describes nitroxide derivatives useful as stabilizers against premature polymerization of vinyl-containing monomers. More particularly, two specific compounds are disclosed : 1-oxyl-2,2,6,6-tetramethylpiperidin-4-yl acetate and 1-oxyl-2,2,6,6-tetramethylpiperidin-4-yl-2-ethylhexanoate.

United States Patent No. 5,545,786 describes the use of selected nitroxide compounds in the prevention of the premature polymerization of vinyl aromatic monomers such as styrene especially in the presence of oxygen. There is no disclosure or suggestion that such nitroxide compounds would be particularly effective in stabilizing acrylic monomers, such as acids, esters or amides, or vinyl acetate or acrylonitrile especially in the presence of water.

United States Patent No. 5,254,760 discloses the use of selected nitroxide compounds in combination with an aromatic nitro compound for stabilizing vinyl aromatic monomers such as styrene. Again, there is no mention of aliphatic vinyl compounds or of the especial effectiveness of some selected nitroxide compounds in preventing the premature polymerization of such aliphatic vinyl monomers in the presence of water.

EP 697,386 generically discloses the use of selected nitroxyl compounds for preventing the premature polymerization of aromatic vinyl monomers such as styrene or aliphatic vinyl monomers such as acrylic monomers. Specifically, this reference teaches that 1-oxyl-4-acetylamino-2,2,6,6-tetramethylpiperidine alone or in combination with p-nitrosophenol or 2-methyl-4-nitrosophenol is effective in stabilizing styrene from premature polymerization. There is no mention that 1-oxyl-4-acetylamino-2,2,6,6-tetramethylpiperidine is used with an aliphatic vinyl monomer alone, and certainly no suggestion that said 1-oxyl-4-acetylamino-2,2,6,6-tetramethylpiperidine would be particularly effective with such aliphatic vinyl monomers in the presence of water.

EP 810,196 discloses the use inter alia of 1-oxyl-2,2,6,6-tetramethyl-4-acetylamino-piperidine in combination with a phosphine, such as triphenylphosphine, or a cobalt compound, such as cobalt acetate, as inhibitors to prevent the polymerization of (meth)acrylic acid or esters thereof. There is no teaching that 1-oxyl-2,2,6,6-tetramethyl-4-acetylaminopiperidine alone would be efficacious for that purpose.

Since, during the processes to produce and purify various ethylenically unsaturated monomers, water is often present during one of the process steps, there is a long felt need for the stable nitroxide inhibitor to be sufficiently water soluble or miscible to remain homogeneous in wet monomer streams and to prevent polymerization in the aqueous phase and yet for the inhibitor to be able to partition to such an extent that it can prevent polymerization in both the aqeuous phase and in the organic monomer phase for inhibition protection throughout the entire process.

The object of this invention is to provide derivatives of 1-oxyl-2,2,6,6-tetramethyl-4-hydroxy-piperidine of sufficient water solubility and the concomitant ability to partition into an organic phase which will prevent the premature polymerization of ethylenically unsaturated monomers in the presence of water.

Another object of this invention to provide novel nitroxide compounds of value in stabilizing unsaturated monomers.

The amount of water present will depend on the specific monomer being stabilized. In the case of monomers of limited compatibility with water such as butyl acrylate, the water content will depend on the amount needed to saturate the ester, only a few percent. On the other hand with water miscible monomers such as acrylic acid, the amount of water possible is theoretically much higher.

The instant invention pertains to a compound of formula III 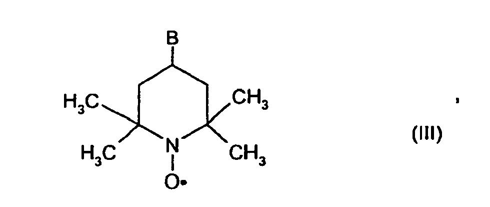 wherein
B is OE₁
E₁ is alkyl of 2 to 6 carbon atoms, substituted by two to three hydroxyl groups or interrupted by one or two oxygen atoms and substituted by said hydroxyl groups, or E₁ is 2-hydroxy-4-oxapentyl with the proviso that E₁ is not 2,3-dihydroxypropyl.

The compounds of formula III, can be prepared with standard methods of organic chemistry. The intermediates are partially commercially available.

The following examples are meant to illustrate the instant invention.

### Example 1 (Reference example)

### 1-Oxyl-2,2,6,6-tetramethyl-4-(2,3-dihydroxypropoxy)piperidine

1.0 g of the compound of Example 3 is heated at 110°C in 50 mL of 5% aqueous sodium hydroxide for six hours. The mixture is extracted with ethyl acetate, and the organic extract is dried and concentrated. The title compound is isolated as a red oil after column chromatography.

### Example 2

### 1-Oxyl-2,2,6,6-tetramethyl4-(2-hydroxy-4-oxapentoxy)piperidine

1.0 g of the compound of Example 3 is heated at 60°C in a solution of 0.25 g sodium methoxide in 50 mL of methanol for six hours. The reaction mixture is then partitioned between water and ethyl acetate. The title compound is isolated as a red oil after column chromatography.

### Method 1

Acrylic acid is distilled to remove any storage stabilizer present. Stock stabilizer solutions (1.5 mg/mL) are prepared in propionic acid. This stock solution is added to the distilled acrylic acid to give a test solution having 5 ppm of total stabilizer. Aliquots of this test solution are then placed into three separate reaction tubes. Each tube is purged with a gas mixture (0.65% oxygen in nitrogen) for ten minutes. The tubes are then sealed and placed in a 110°C oil bath. The tubes are watched till the appearance of visible polymer formation is observed as a precipitate. Failure times are reported as an average of at least three tubes.

### Method 2

Test solutions are prepared as in Method 1 except that the stock stabilizer solution is prepared at 0.75 mg/mL giving a test solution with 2.5 ppm of total stabilizer. Aliquots (1 mL) of the test solution are placed into three separate reaction tubes. To each tube is added 0.5 mL of toluene and 0.5 mL of distilled water. Each tube is purged as described in Method 1 and then sealed. The tubes are placed in a 90°C oil bath and heated till visible polymer is observed as a precipitate. Failure times are reported as an average of at least three tubes.

### Example 3

Procedure of Method 2 where water is present in the acrylic acid is applied.

**Table 2**

| Stabilization of Aqueous Acrylic Acid | |
|---|---|
| Compound* of Example (5 ppm by weight) | Time to Onset of Polymerization minutes |
| none | 30 |
| A | 240 |
| B | 130 |
| Example 1 | 320 |
| Example 2 | 350 |

| | |
|---|---|
| *A is bis(1-oxyl-2,2,6,6-tetramethylpiperidin-4-yl) sebacate. B is 1-oxyl-2,2,6,6-tetramethylpiperidin-4-yl octanoate. | |

## Claims

1. A compound of formula III 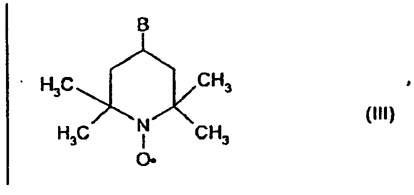 wherein
B is OE₁
E₁ is alkyl of 2 to 6 carbon atoms substituted by two to three hydroxyl groups or interrupted by one or two oxygen atoms and substituted by said hydroxyl groups,
or E₁ is 2-hydroxy-4-oxapentyl with the proviso that E₁ is not 2,3-dipydroxypropyl.

## Patentansprüche

1. Verbindung der Formel III 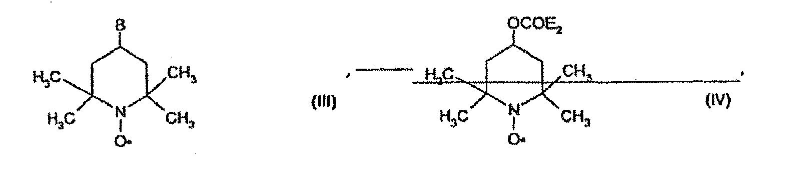 worin
B für OE₁ steht;
E₁ für Alkyl mit 2 bis 6 Kohlenstoffatomen, das durch zwei bis drei Hydroxylgruppen substituiert oder durch ein oder zwei Sauerstoffatome unterbrochen und durch die Hydroxylgruppen substituiert ist, steht oder E₁ für 2-Hydroxy-4-oxapentyl steht; mit der Maßgabe, daß E₁ nicht für 2,3-Dihydroxypropyl steht.

## Revendications

1. Composé de formule III 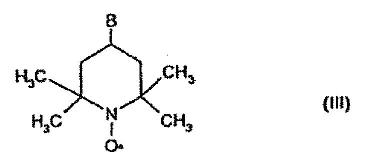 dans laquelle
B est OE₁
E₁ est alkyle de 2 à 6 atomes de carbone, substitué par deux à trois groupements hydroxy ou interrompu par un ou deux atomes d'oxygène et substitué par lesdits groupements hydroxy, ou E₁ est 2-hydroxy-4-oxapentyle, à condition que E₁ ne soit pas 2,3-dihydroxypropyle.
